# EUROPÄISCHE PATENTSCHRIFT

(11) **EP 1 495 772 B1**
(45) Veröffentlichungstag und Bekanntmachung des Hinweises auf die Patenterteilung: **12.11.2008**
(21) Anmeldenummer: 04010102.4
(22) Anmeldetag: 28.04.2004
(51) Int. Cl.: A61L 12/08, A61L 12/10, A61L 12/14, C11D 3/00

(54) **Verwendung von Taurolidin, Taurultam oder Mischungen davon als antimikrobieller Zusatz in Zubereitungen zur Behandlung, Reinigung, Aufbewahrung und zum Konditionieren von Kontaktlinsen**
Use of taurolidine, taurultame or mixtures thereof as antimicrobial additive in preparations for treating, cleaning, storing and conditioning of contact lenses
Utilisation de taurolidine, taurultame et de leurs mélanges comme additifs antimicrobiens dans de compositions pour le traitment, nettoyage, rangement et conditionnement de lentilles de contact

(30) Priorität: 11.07.2003 DE 10331557
(43) Veröffentlichungstag der Anmeldung: 12.01.2005
(73) Patentinhaber: HERDEIS, Claus, 80809 München (DE); Weis, Christian Edwin, 97297 Waldbüttelbrunn (DE)
(72) Erfinder: HERDEIS, Claus, 80809 München (DE); Weis, Christian Edwin, 97297 Waldbüttelbrunn (DE)
(74) Vertreter: Andrae, Steffen

(56) Entgegenhaltungen:
- EP-A- 0 958 836
- EP-A- 1 293 126
- WO-A-86/05401
- WO-A-95/27515
- WO-A-99/24543
- H. OGUZ ET AL.: CURRENT EYE RESEARCH, Bd. 21, Nr. 5, 2000, Seiten 851-855, XP001182658

## Beschreibung

Die Erfindung betrifft die Verwendung von Taurolidin, Taurultam oder Mischungen davon als antimikrobielles Mittel in Lösungen zur Behandlung, Reinigung, Aufbewahrung und zum Conditioning von Kontaktlinsen.

Das Risiko von Kontaktlinsenbenutzern, an infektiösen Hornhautentzündungen zu erkranken, ist im Vergleich zu Brillenträgern um das etwa 80fache erhöht (DER SPIEGEL 11/2003, S. 171). Hygieneempfehlungen werden teils aus Bequemlichkeit und teils aus Unkenntnis nicht befolgt. Gerade im Zeitalter der 30 Tages-Linse wird aufgrund der kurzen Nutzungsdauer die tägliche Desinfektion der Kontaktlinse mehr und mehr vernachlässigt. Das Auge wird durch den Einfluß der Linse (Ansteigen der Temperatur um 2°C, Sauerstoffmangel, kleinere Verletzungen beim Einsetzen und Herausnehmen der Linse) zusätzlich gestreßt. Es muß daher eine gründliche Desinfektion der Kontaktlinsen erfolgen, um die Wahrscheinlichkeit von Infektionen im Zusammenhang mit dem Tragen der Kontaktlinse zu reduzieren. Es ist dabei bekannt, dass die Effektivität von Desinfektionsmitteln nach Ausbildung eines Biofilms drastisch reduziert wird (vgl. R.M. Donlan, Emerging Infectious Diseases, Vol.8, No.9, Sept 2002).

Nach neuesten Untersuchungen wirken jedoch gerade die marktüblichen Desinfektionsmitteln zu schwach, um Keime auf den Kontaktlinsen und in den Aufbewahrungsbehältern abzutöten. So konnten in einem konkreten Fall die als äußerst gefährlich einzustufenden Pseudomonas Keime nicht nur im Auge, sondern auch auf der Kontaktlinse und in der Aufbewahrungslösung nachgewiesen werden.

Viele der Problemkeime sind bereits gegen die gängigen Mittel resistent. Es wäre deshalb erforderlich, auf neue und wirksamere Desinfektionsmittel zurückzugreifen. Andererseits greifen gerade starke Reiniger das Auge und den empfindlichen Kunststoff der weichen Kontaktlinsen an. Dies ist der Grund, warum Hersteller auf schonendere, damit aber riskantere und weniger wirksame Mittel ausweichen.

Es ist bekannt, in wirkstoffhaltigen ophthalmischen Arzneimitteln als Konservierungsmittel quaternäre Ammoniumsalze zu verwenden (vgl. z.B. EP-A-306 984 oder EP-A-242 328). Aus der großen Zahl der als quaternäre Ammoniumsalze in Frage kommenden Konservierungsmittel haben zur Konservierung von opthalmischen Zubereitungen jedoch - offenbar aufgrund der vielfältigen Anforderungen, die erfüllt sein müssen - nur ganz wenige eine praktische Verwendung gefunden, und zwar insbesondere die drei in EP-A-306 984 explizit genannten Substanzen Cetyltrimethylammoniumbromid, Cetylpyridiniumchlorid und Benzalkoniumchlorid (vgl. auch EP-A-242 328). Das Konservierungsmittel der Wahl für Augenarzneimittel ist zur Zeit ganz eindeutig das letztgenannte Benzalkoniumchlorid (s. z.B. EP-A-306 984, Seite 2, Zeilen 31-33), welches die folgende Struktur besitzt: N-Benzyl-N-(C8-C18-alkyl)--N,N-dimethylammoniumchlorid. Weitere Substanzen zur Konservierung von opthalmischen Arzneimitteln sind beispielsweise Chlorhexidin, Thiomersal und Chlorobutanol.

In ophthalmischen Arzneimitteln erfüllen die Konservierungsmittel in erster Linie den Zweck, einen Befall des Mittels durch Mikroorganismen und deren Ausbreitung zu verhindern. Während die genannten quaternären Ammoniumsalze diesen Zweck erfüllen können, werden an antimikrobielle Mittel in Lösungen zur Behandlung, Reinigung, Aufbewahrung und zum Conditioning von Kontaktlinsen insofern höhere Anforderungen gestellt, als die antimikrobiellen Mittel auch in der Lage sein müssen, Mikroorganismen, die z.B. aus einem infizierten Auge stammen und an einer Kontaklinse haften, zuverlässig abzutöten und eine sterile, saubere Oberfläche zu hinterlassen.

Wegen der schwierigen Balance zwischen der gewünschten antimikrobiellen und der Reinigungs-Wirkung, der erforderlichen Unschädlichkeit für das Material der Kontaktlinsen und der Verträglichkeit der Zubereitungen/Lösungen bzw. Lösungsreste mit dem Auge wurde noch keine Problemlösung gefunden, die als optimal zu bezeichnen wäre, d.h. eine Zubereitung, die eine hohe antimikrobiellen Sicherheit im Hinblick auf die verschiedensten möglichen Infektionskeime bei einer gleichzeitig geringen Aggressivität gegenüber dem Kontaktlinsenmaterial aufweist und die das Auge nicht reizt oder schädigt.

Es besteht daher weiterhin ein Bedarf nach einem antimikrobiellen Mittel, das Lösungen zur Behandlung, Reinigung, Aufbewahrung und zum Conditioning von Kontaktlinsen zugesetzt werden kann und derartige Lösungen nicht nur sicher gegen einen Befall durch Mikroorganismen schützt, sondern auch eine zuverlässige kontinuierliche Desinfektion von Kontaktlinsen während ihrer Gebrauchsdauer gewährleistet.

Die genannte Aufgabe wird erfindungsgemäß dadurch gelöst, dass als antimikrobieller Wirkzusatz zu Lösungen zur Behandlung, Reinigung, Aufbewahrung und zum Conditioning von Kontaktlinsen Taurolidin, Taurultam, bzw. Mischungen daraus verwendet werden.

Es werden somit Lösungen zur Behandlung, Reinigung, Aufbewahrung und Conditioning von Kontaktlinsen verwendet, die das antimikrobielle Chemotherapetikum Taurolidin als Desinfektionsmittel und Konservierungsmittel enthalten, die sich durch eine bisher nicht gekannte Kombination aus Augenverträglichkeit/Desinfektionsqualität auszeichnen.

Taurolidin ist ein 1,1-Dioxo-perhydro-1,2,4-thiadiazin--Derivat und eine seit über 30 Jahren bekannte Substanz. Seine Herstellung ist beschrieben in der CH-A-482 713. Taurolidin wird durch die allgemeine Formel (I) beschrieben 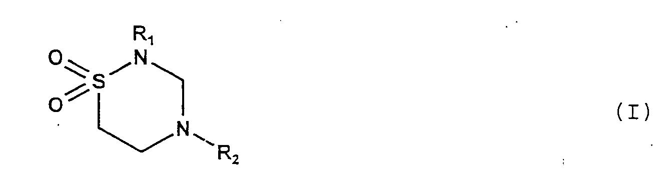 in der R¹ für H steht und R² ein Rest der allgemeinen Formel (II) ist: 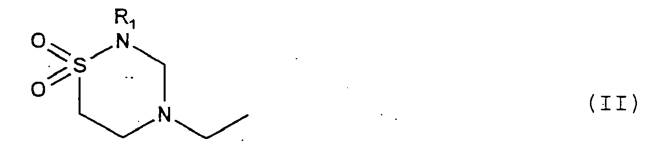 Die verwandte Verbindung Taurultam ist eine Verbindung der allgemeinen Formel (I), in der R² für Wasserstoff steht.

Ein modernes vorteilhaftes Verfahren zur Herstellung von Taurolidin bzw. ggf. auch Taurultam ist beschrieben in der EP-A-0 863 133.

Taurolidin fand bisher fast ausschließlich Anwendung in der Humanmedezin. So beschreibt beispielsweise EP-A-0 253 662 die Verwendung von Taurolidin in Form einer wäßrigen Lösung zur parenteralen Verabreichung bei chirurgischen Eingriffen gegen Infektionen durch Bakterien oder bakterielle Toxine. WO-A-92/000743 beschreibt ein Verfahren zur Behandlung oder Prophylaxe von Tumoren durch Verabreichung einer effektiven Dosis von Taurolidin und/oder Taurultam. In der DE-A-35 33 612 wird die Verwendung von Taurolidin als die Blutgerinnung hemmendes Mittel beschrieben. WO-A-94/03174 beschreibt ein Taurolidin enthaltendes zahnmedizinisches Mittel zur Behandlung von beispielsweise Parodontitis. Die einzige diesseits bekannte Anwendung von Taurolidin am Auge ist die als antimikrobieller Wirkstoff in einem Arzneimittel für die topische Anwendung zur Verminderung von potentiell augenschädlichen Mikroorganismen (H. Oguz et al., Current Eye Research, 2000, Vol.21, No.5, pp.851-855). Bei der beschriebenen Anwendung von Taurolidin wurden keine Irritationen am Auge festgestellt. In vitro-Verwendungen von Taurolidin, die eher als Verwendungen als Oberflächen-Desinfektionsmittel und/oder Konservierungsmittel zu bezeichnen wären, sind unbekannt.

Die Wirkungsweise von Taurolidin gegen Mikroorganismen bzw. Bakterien beruht auf der Übertragung von Methylolgruppen auf die Hydroxyl- oder Aminogruppen von Bakterienzellwänden. Die im Vergleich zu Antibiotika relativ hohe minimale Hemmkonzentration von Taurolidin gegen Staphylococcus aureus von 0.3-0.6 mg/ml ließ zunächst keine besondere Wirksamkeit bei der Verwendung zur Desinfektion von Kontaktlinsen erwarten.

Daneben hat die Substanz antiendotoxische und antiinflammatorische Eigenschaften. Aufgrund des Abbaus des Taurolidins letztendlich zur biogenen Aminosäure Taurin erklärt sich vermutlich die sehr geringe Toxizität und gute Gewebeverträglichkeit der Substanz. Resistenzentwicklungen sind bisher nicht bekannt, was von außerordentlicher Wichtigkeit auch für die Verwendungen gemäß der vorliegenden Erfindung ist.

Überraschenderweise wurde gefunden, dass Taurolidin, Taurultam und Mischungen daraus zur Reinigung und Desinfektion von Kontaktlinsen außerordentlich wirksam sind. Diese Substanzen besitzen eine bisher unbekannte und einzigartige Kombination aus Augenverträglichkeit einerseits und äußerst wirksamer Desinfektion andererseits. Die beobachtete Wirkung geht hierbei über die konservierende Wirkung weit hinaus, wie sie für Nahrungsmittel und pharmazeutische und kosmetische Produkte beschrieben ist in der Französischen Patentanmeldung FR-A-2 771 928, da nicht nur eine Konservierung im Sinne einer Verhinderung eines Befalls durch Mirkoorganismen beobachtet wird, sondern sogar eine vollständige Reduktion von bereits vorhandenen Keimen und damit eine ideale Desinfektion während der Aufbewahrungszeit stattfindet. Die Entstehung eines Biofilms auf der Kontaktlinse wurde nicht beobachtet.

Zur sicheren vollständigen Desinfektion von Kontaktlinsen ist ein Kontakt zwischen der taurolidinhaltigen Zubereitung/Lösung und den Kontaklinsen im Sinne einer Aufbewahrung von mindestens 30 min notwendig. Dies unterscheidet die genannten Substanzen von klassischen Desinfektionsmitteln, für die gefordert wird, dass sie innerhalb von 2-3 min wirken. Als Chemotherapeutika besitzen die genannten Substanzen nicht die Nachteile von Antibiotika, beispielsweise die Ausbildung von Resistenz. Bei topischer Applikation auf dem Auge wird überraschenderweise keinerlei Reizwirkung ausgelöst, und es wird auch keinerlei Beschädigung von Kontaktlinsen verursacht.

Die Konzentration an Taurolidin, Taurultam oder Mischungen davon in den erfindungsgemäßen Lösungen beträgt vorzugsweise 0.005 bis 10.00 Gew.-%, bevorzugt 0.01-5.0 Gew.-%, jeweils bezogen auf das Gesamtgewicht der Zubereitungen.

Die jeweils einzusetzende Art und Menge des jeweils eingesetzten Trägerstoffs oder Emulgators kann in Abhängigkeit von der Art des jeweiligen Produktes vom Fachmann durch einfaches Ausprobieren leicht ermittelt werden.

Nachfolgend wird die Erfindung anhand derzeit bevorzugter Ausführungsbeispiele näher erläutert.

### Beispiel 1:

Reinigungslösung für Kontaktlinsen

| Bestandteil | Menge |
|---|---|
| Taurolidin | 0,200 g |
| Natriumchlorid | 0,36 g |
| Na₂HPO₄ | 0,73 g |
| NaH₂PO₄.H₂O | 0,21 g |
| Dinatrium-EDTA | 0,10 g |
| Varox^{®} TM 36 | 51,00 ml |
| Triton^{®} TM X-100 | 0,33 ml |
| Cellosize^{®} TM QP-40 HEC | 1,50 g |
| Demineralisiertes Wasser ad | 100,00 ml |

Bei den mit Handelsnamen/Markennamen bezeichneten Produkten Varox® TM 36, Triton® TM X-100 und Cellosize® TM QP-40 HEC handelt es sich um Handelsprodukte der folgenden Art:
- Varox TM 36: - ein Siliconöl für pharmazeutische Zubereitungen
- Triton® TM X-100: - nichtionisches Tensid; Octylphenoxypolyethoxyethanol (Mallinkroth Baker, Inc., Phillipsburg, NJ 08865, USA)
- Cellosize® TM QP-40: HEC - Hydroxyethylcellulose (Dow Chemicals)

### Beispiel 2:

Konditionierungslösung für gasdurchlässige und harte Kontaktlinsen

| Bestandteil | Menge |
|---|---|
| Taurolidin | 0,200 g |
| Vinol^{®} TM 350 PVA | 0,500 g |
| Plasdone^{®} K-90 PVP | 1,000 g |
| Na₂HPO₄ | 0,720 g |
| NaH₂PO₄.H₂O | 0,220 g |
| Natriumchlorid | 0,440 g |
| Dinatrium-EDTA | 0,100 g |
| Demineralisiertes Wasser | ad 100,00 ml |

Bei den mit Handelsnamen/Markennamen bezeichneten Produkten Vinol^{®} TM 350 PVA und Plasdone^{®} K-90 PVP handelt es sich um Handelsprodukte der folgenden Art:
- Vinol^{®} TM 350 PVA -: Polyvinylalkohol
- Plasdone^{®} K-90 PVP -: Polyvinylpyrrolidon (ISP)

## Patentansprüche

1. Verwendung von Taurolidin, Taurultam oder Mischungen davon als antimikrobieller Zusatz zu Zubereitungen zur Behandlung, Reinigung, Aufbewahrung und zum Conditioning von Kontaktlinsen

2. Verwendung nach Anspruch 1, wobei die Konzentration an Taurolidin und/oder Taurultam 0.005 bis 10.00 Gew.-%, jeweils bezogen auf das.Gesamtgewicht der Zubereitungen, beträgt.

3. Verwendung nach Anspruch 2, wobei die Konzentration 0.01 bis 5.00 Gew.-%, jeweils bezogen auf das Gesamtgewicht der Zubereitungen, beträgt.

## Claims

1. Use of taurolidine, taurultame or mixtures thereof as antimicrobial additive to preparations for the treatment, cleaning, storage and conditioning of contact lenses.

2. Use according to Claim 1, wherein the concentration of taurolidine and/or taurultame is 0,005 to 10,00 % by weight, based on the total weight of the preparation.

3. Use according to Claim 2, wherein the concentration is 0,01 to 5,00 % by weight, based on the total weight of the preparation.

## Revendications

1. Utilisation de taurolidine, de taurultame ou de leurs mélanges comme additif antimicrobien pour des préparations pour le traitement, le nettoyage, la conservation et le conditionnement de lentilles de contact.

2. Utilisation selon la revendication 1, dans laquelle la concentration en taurolidone et/ou en taurultame se situe dans l'intervalle allant de 0,005 à 10,00% en poids, chaque fois sur base du poids total des préparations.

3. Utilisation selon la revendication 2, dans laquelle la concentration se situe dans l'intervalle allant de 0,01 à 5,00% en poids, chaque fois sur base du poids total des préparations.
